# EUROPEAN PATENT SPECIFICATION

(11) **EP 3 213 725 B1**
(45) Date of publication and mention of the grant of the patent: **25.03.2020**
(21) Application number: 17158642.3
(22) Date of filing: 01.03.2017
(51) Int. Cl.: A61F 9/02

(54) **VISOR MASK**
VISIERMASKE
MASQUE DE VISIÈRE

(30) Priority: 01.03.2016 IT UB20161204
(43) Date of publication of application: 06.09.2017
(73) Proprietor: ETHEN S.R.L., 20842 Besana Brianza (MB) (IT)
(72) Inventor: ROCCA, Davide Antonio, 23884 Castello Brianza (LC) (IT); ROCCA, Fabio Enrico, 20842 Besana Brianza (MB) (IT)
(74) Representative: Borsano, Corrado

(56) References cited:
- WO-A1-2005/121689
- DE-A1-102005 051 575
- US-A- 4 686 712
- US-A1- 2003 081 169
- US-A1- 2013 014 316
- US-B1- 7 055 521

## Description

### Field of the invention

The present invention relates to the field of vision facilitation accessories, more specifically to a visor mask.

### Background art

Visor masks are known in the art which are to be worn on the front part of the face, over the eyes, for a number of applications, especially for sporting or racing activities, such as skiing and motorcycling, or for accident prevention or building site applications.

Said known masks are normally made up of parts joined together by complex systems, including joints of various shapes, bayonet fasteners or magnets.

The use of said known masks poses problems of variable nature. For example, it is difficult to quickly replace the visor or other component parts, e.g. in cold weather conditions or in dangerous environments, when the user is wearing protective gloves that cannot be removed, but said parts nevertheless need to be replaced.

Other problems derive, for example, from the excessive weight of some of said known masks and parts thereof, or from the excessive cost of replacement parts, so that one may finally decide to replace the entire mask instead of only replacing the worn-out parts.

US 4 686 712 A discloses a visor mask comprising a closed-type frame with a closed contour supporting structure, a visor applied to the front of the frame, and a spongy layer applied to the rear part of the frame.

WO 2005/121689 A1 and US 2013/014316 A1 describe a visor mask comprising a closed-type frame with a closed contour supporting structure, a visor applied to the front part of the frame, and which also surrounds the part of the face including the nose, in which air filters are present.

### Summary of the invention

It is therefore the object of the present invention to propose a visor mask which can overcome all of the above-mentioned problems.

The invention is defined in the independent claim. The basic idea of the present invention is to join together the component parts of the visor mask by using Velcro.

The present invention relates to a visor mask comprising:
- a frame having a closed-contour bearing structure;
- a visor or lens applied to the front part of the frame;
- a foam layer applied to the rear part of the frame;
- one or more aeration filters, applied to the lateral edge of the frame,
- one or more aeration filters, applied to the lateral edge of the frame,
the mask comprising Velcro, and one or more of said visor or lens, foam layer, one or more aeration filters being removably applied to the frame by means of said Velcro, said Velcro comprising two parts, one part being applied to said frame and the other part being applied to one or more of said visor or lens, foam layer, one or more aeration filters in locations or areas matching the areas of application to the frame.

In particular, the present invention relates to a visor mask as set out in detail in the claims, which are an integral part of the present description.

### Brief description of the drawings

Further objects and advantages of the present invention will become apparent from the following detailed description of a preferred embodiment (and variants) thereof referring to the annexed drawings, which are only supplied by way of non-limiting example, wherein:

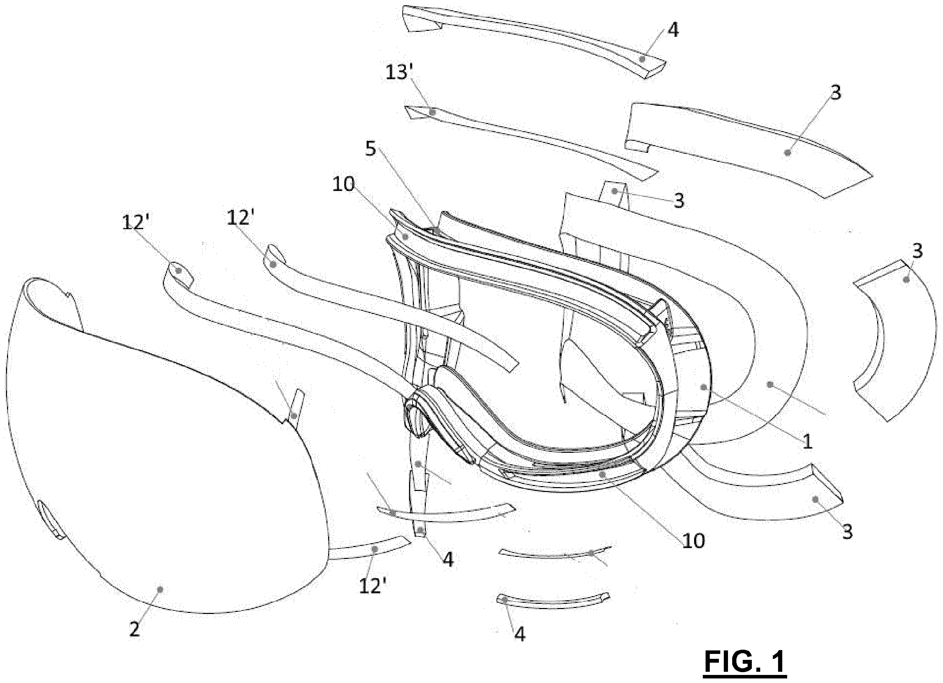

Figure 1 shows a first exploded view of the component parts of the mask of the present invention;

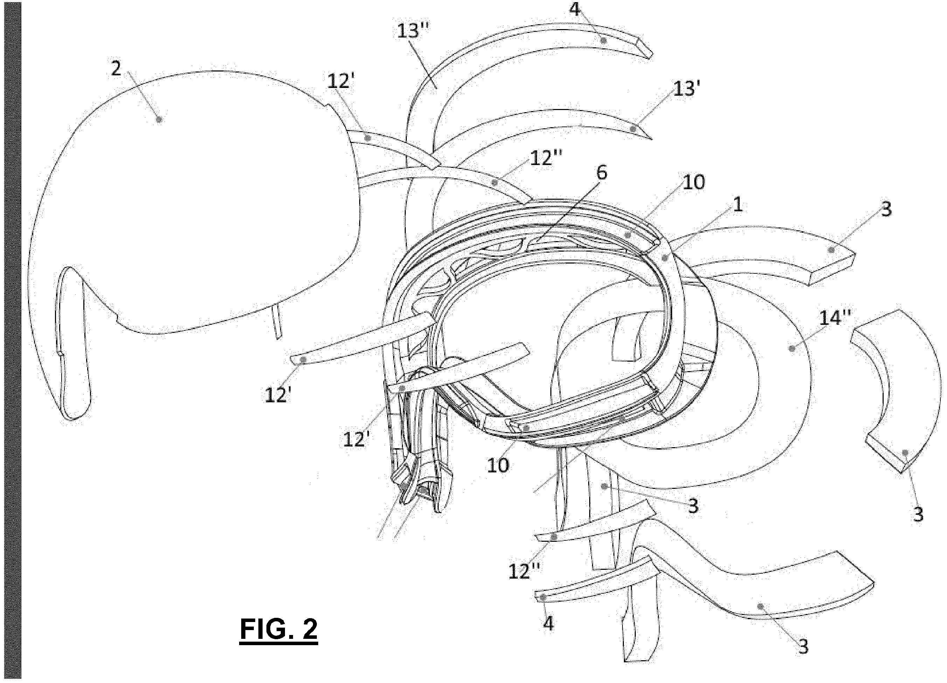

Figure 2 shows a second exploded view of the component parts of the mask;

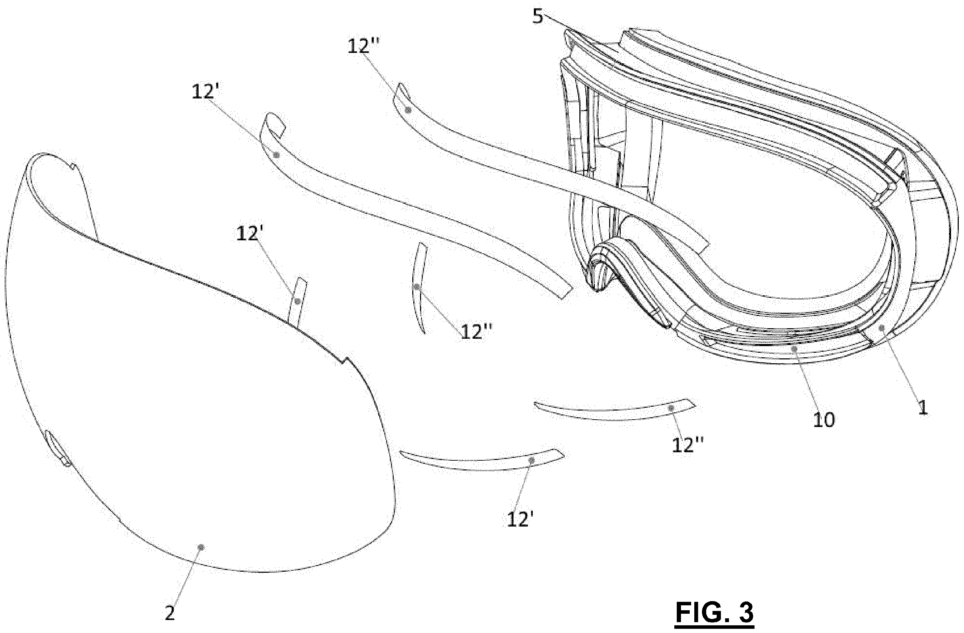

Figure 3 shows an exploded view of the component parts of the front visor of the mask;

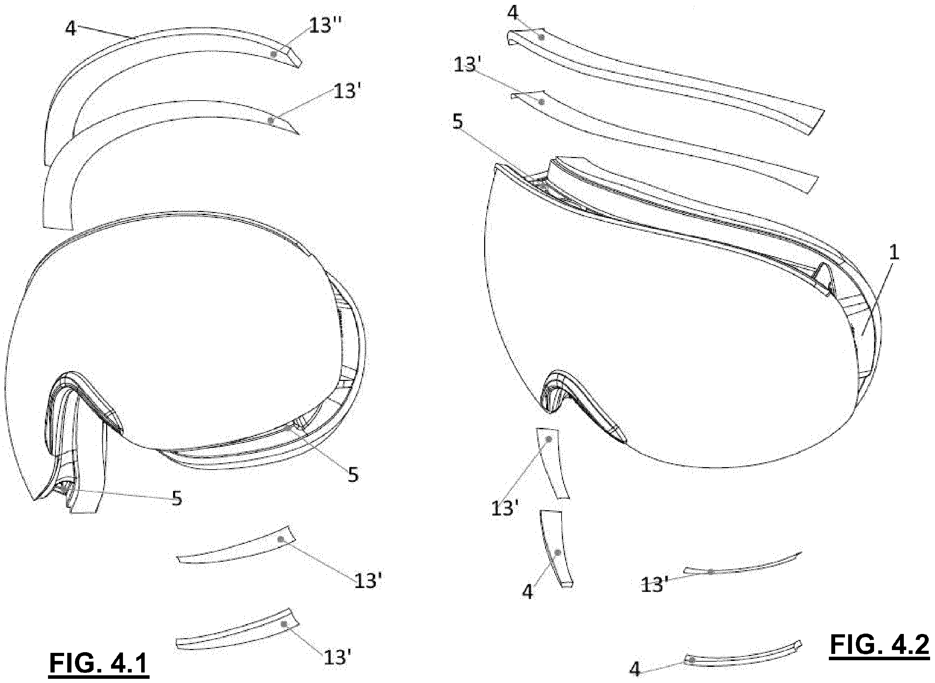

Figures 4.1 and 4.2 show two exploded views of the component parts of the aeration filters of the mask;

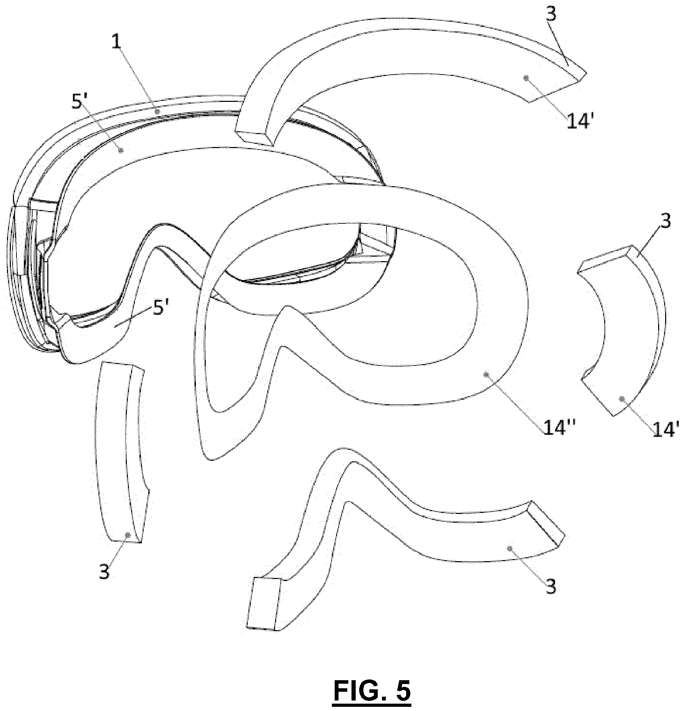

Figure 5 shows an exploded view of the component parts of the rear foam layer of the mask;

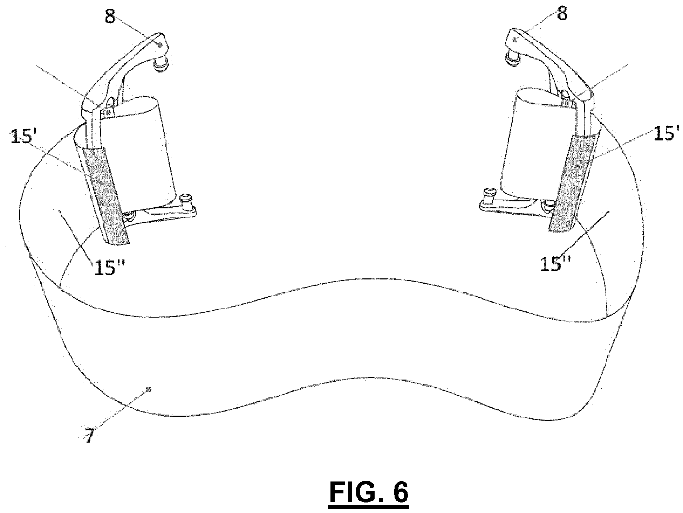

Figure 6 shows a view of the component parts of the elastic band of the mask.

In the drawings, the same reference numerals and letters identify the same items or components.

### Detailed description of some embodiments of the invention

With reference to the annexed drawings, the visor mask of the invention is essentially made up of the following main parts.
- A frame 1 having a closed-contour bearing structure, preferably made from elastic material, e.g. polyurethane; preferably, its shape is adapted for adhering to the face of the person wearing the mask in the part in front of the eyes, leaving the nose unaffected; the elasticity thereof also allows a certain degree of shock energy absorption.
- A visor or lens 2 with a curvilinear screen, made from elastic transparent material, e.g. plastic material, preferably as one piece, and adapted to adhere and be removably fixed to the front part of the frame.
- A foam layer 3 made of compressible and re-expandable soft material, e.g. foam or sponge, adapted to adhere and be removably fixed to the rear part of the frame, and also adapted to directly adhere to the face of the person wearing the mask, so as to improve adhesion, by adapting the frame to the shape of the face; normally the rear contour is also useful for absorbing the sweat emission from the face.
- One or more aeration filters 4 with a strip-like or band-like structure, adapted to adhere and be removably fixed to the lateral edge 5 of the frame; the lateral edge 5 has a grid-like structure 6 with aeration apertures to allow perspiration. The filters can also control perspiration and maintain clean conditions inside the mask, i.e. on the face. This control can be adjusted by selecting the filtering rate of the filters.
- An optional elastic strip or band 7, the terminal parts thereof being wound around and removably fixed to two lateral extensions or claws 8 of the frame; The lateral extensions or claws 8 are preferably rigidly connected to the frame or swivelling about pins; the elastic band is adapted to connect the mask firmly to the face by extending on the back of the head, whether directly or on a helmet covering the person's head. The elastic band 7 may even be absent, if the mask is applied differently to the person's face, e.g. by adhesion to a protective helmet. In accordance with a main aspect of the present invention, one or more of the above-mentioned parts of the mask (visor or lens 2, contour 3 made of soft material, aeration filters 4, elastic band 7) are joined to the frame 1 in a removable manner by means of Velcro. Velcro is a light, easy-to-use, strong and durable elastic material, which can resist to critical climatic conditions; its tightening capability is promoted by the overall lightness of the mask.

As is *per se* known, Velcro is made up of two strip-like or band-like parts, the inner faces of which are made to adhere to each other in a removable manner to ensure adhesion or connection.

The outer face of one Velcro part is adapted to be fixed or glued to specific contour parts of the frame, whereas the outer face of the other Velcro part is adapted to be fixed or glued to corresponding contours or parts of the other components of the mask.

The inner faces of the two strip-like or band-like Velcro parts that must adhere to each other have surfaces with specific roughness characteristics to ensure removable adhesion, such as, for example, double-hook, mushroom or cap-like reliefs, or one of the two faces has a web-like weft into which the rigid hooks of the other part can engage.

Preferably, the front contour 10 of the frame 1 has a cavity adapted to house the Velcro strip 12" prearranged for attaching the visor 2. The cavity 10, including the Velcro strip, may be present on most of the front contour, possibly leaving a lateral part 11 without Velcro to facilitate the manual replacement of the visor, into which lateral part a person can insert a finger to detach the visor. To the contour of the visor 2, a corresponding Velcro part 12' is attached.

The grid-like structure 6 of the lateral edge 5 of the frame may only be provided on some portions of the lateral edge, e.g. the upper part and the lower part thereof, to which one Velcro part 13' is applied, whereas the other Velcro part 13" is applied to the corresponding aeration filter 4.

The rear foam layer 3 may be either made as one piece or divided into parts or zones, even having different thickness or consistency or density or thermal characteristics or number of component layers, for the purpose of optimizing the "fitting" characteristics for better compliance with the conformation of the face, so that even just one of such parts can be replaced, e.g. near the nose, the temples or the forehead. The foam layer 3 is applied to the rear part of the frame by means of Velcro, one part 14' of which is applied to the foam layer and the other part 14"is applied to the rear edge 5' of the frame.

The terminal parts of the elastic band 7 are wound and removably fixed around two lateral extensions or claws 8 of the frame by application of Velcro, e.g. one part thereof 15' being applied to the outer edges of the elastic band and the other part thereof 15" being applied to the inner wall of the elastic band in a sufficiently rearward position.

Thus, the use of Velcro allows speeding up and facilitating the replacement of the component parts of the mask, and contributes to reducing the weight of the mask considerably. It is not necessary to remove the mask in order to replace the visor, since the front part is clear and hence easy to manipulate and release, even while wearing gloves.

The modified mask of the invention is particularly suited to applications such as sporting or racing activities (skiing, motorcycling), target shooting, or for accident prevention or building site applications. It is also a category 1 protection device that provides some ballistic resistance and is suitable for paint use.

The above-described non-limiting example of embodiment may be subject to variations without departing from the protection scope of the present invention, including all equivalent designs known to a man skilled in the art.

The elements and features shown in the various preferred embodiments may be combined together without however departing from the protection scope of the present invention.

From the above description, those skilled in the art will be able to produce the object of the invention without introducing any further construction details.

## Claims

1. Visor mask comprising:
- a frame (1) having a closed-contour bearing structure;
- a visor or lens (2) applied to the front part of the frame;
- a foam layer (3) applied to the rear part of the frame;
- one or more aeration filters (4),
the visor mask being **characterized in that**:
said one or more aeration filters (4) have a strip-like or band-like structure and are adapted to adhere and to be removably fixed to the lateral edge (5) of the frame, and **in that** it comprises Velcro (registered trademark for a hook-and-loop fastener);
and **in that** said one or more aeration filters (4) are removably applied to the frame by means of said Velcro, the latter comprising two parts, one part being applied to said frame and the other part being applied to said one or more aeration filters (4) in locations or areas matching the areas of application to the frame.

2. Visor mask according to claim 1, comprising an elastic strip or band (7), the terminal parts of said elastic band being wound around two lateral extensions or claws (8) of the frame and being removably secured by means of said strip-like or band-like parts.

3. Visor mask according to claim 1, wherein said frame (1) is made of elastic material, in particular, polyurethane, and its shape adapts itself by adhesion to the face of the person wearing the mask in the part in front of the eyes, leaving the nose unaffected.

4. Visor mask according to claim 1, wherein said visor or lens (2) is a curvilinear screen made as one piece from elastic or plastic transparent material.

5. Visor mask according to claim 1, wherein at least a part of said front part of the frame has a cavity (10) adapted to receive said strip-like or band-like parts, the remaining part of said front part bearing no Velcro, so that said visor or lens (2) can be easily replaced.

6. Visor mask according to claim 1, wherein said lateral edge (5) of the frame has a grid-like structure (6) with apertures.

7. Visor mask according to claim 1, wherein said foam layer (3) is made of compressible and re-expandable soft material, either as one piece or divided into parts or zones having equal or different thickness or consistency or density or thermal characteristics or number of component layers.

## Patentansprüche

1. Visiermaske aufweisend:
- einem Rahmen (1) mit einer Tragstruktur mit geschlossener Kontur;
- ein Visier oder eine Linse (2), die auf den vorderen Teil des Rahmens aufgebracht ist;
- eine Schaumstoffschicht (3), die auf den hinteren Teil des Rahmens aufgebracht ist;
- einen oder mehrere Belüftungsfilter (4),
wobei die Visiermaske **dadurch gekennzeichnet** ist:
der eine oder die mehreren Belüftungsfilter (4) eine streifen- oder bandartige Struktur aufweisen und dazu geeignet sind, an der Seitenkante (5) des Rahmens zu haften und lösbar befestigt zu werden, und indem es Velcro (eingetragene Marke für einen Klettverschluss) aufweist;
und dass der eine oder die mehreren Belüftungsfilter (4) mittels des genannten Velcro abnehmbar am Rahmen angebracht sind,
wobei Velcro aus zwei Teilen besteht, wobei ein Teil an dem Rahmen und der andere Teil an dem oder den Belüftungsfiltern (4) an Stellen oder Bereichen angebracht wird, die den Anwendungsbereichen des Rahmens entsprechen.

2. Visiermaske nach Anspruch 1, aufweisend einen elastischen Streifen oder ein elastisches Band (7), wobei die Endteile des elastischen Bandes um zwei seitliche Verlängerungen oder Klauen (8) des Rahmens gewickelt sind und mittels der streifen- oder bandartigen Teile abnehmbar befestigt sind.

3. Visiermaske nach Anspruch 1, wobei der Rahmen (1) aus elastischem Material, insbesondere Polyurethan, hergestellt ist und seine Form sich durch Haftung am Gesicht der die Maske tragenden Person in dem Teil vor den Augen anpasst, wobei die Nase unbeeinflusst bleibt.

4. Visiermaske nach Anspruch 1, wobei das genannte Visier oder die genannte Linse (2) ein krummliniger Schirm ist, der in einem Stück aus elastischem oder plastischem, transparentem Material hergestellt ist.

5. Visiermaske nach Anspruch 1, wobei mindestens ein Teil des Vorderteils des Rahmens einen Hohlraum (10) aufweist, der zur Aufnahme der streifen- oder bandartigen Teile geeignet ist, wobei der verbleibende Teil des Vorderteils kein Velcro trägt, so dass das Visier oder die Linse (2) leicht ausgewechselt werden kann.

6. Visiermaske nach Anspruch 1, wobei der seitliche Rand (5) des Rahmens eine gitterartige Struktur (6) mit Öffnungen aufweist.

7. Visiermaske nach Anspruch 1, wobei die Schaumstoffschicht (3) aus komprimierbarem und wieder expandierbarem weichen Material hergestellt ist, entweder als ein Stück oder in Teile oder Zonen mit gleicher oder unterschiedlicher Dicke oder Konsistenz oder Dichte oder thermischen Eigenschaften oder Anzahl von Komponentenschichten unterteilt ist.

## Revendications

1. Masque à visière comprenant :
- un cadre (1) ayant une structure porteuse à contour fermé ;
- une visière ou un verre (2) appliqué sur la partie avant du cadre ;
- une couche de mousse (3) appliquée sur la partie arrière du cadre ;
- un ou plusieurs filtres d'aération (4),
le masque à visière étant **caractérisé en ce que** :
lesdits un ou plusieurs filtres d'aération (4) ont une structure de type bande ou de type ruban et sont adaptés pour adhérer et être fixés de manière amovible au bord latéral (5) du cadre, et **en ce qu'**il comprend un Velcro (marque déposée pour une fermeture auto-agrippante) ;
et **en ce que** lesdits un ou plusieurs filtres d'aération (4) sont appliqués de manière amovible au cadre au moyen dudit Velcro, ce dernier comprenant deux parties, une partie étant appliquée sur ledit cadre et l'autre partie étant appliquée sur lesdits un ou plusieurs filtres d'aération (4) dans des emplacements ou des zones correspondant aux zones d'application sur le cadre.

2. Masque à visière selon la revendication 1, comprenant une bande ou un ruban élastique (7), les parties terminales dudit ruban élastique étant enroulées autour de deux extensions ou griffes latérales (8) du cadre et étant arrimées de manière amovible au moyen desdites parties de type bande ou de type ruban.

3. Masque à visière selon la revendication 1, dans lequel ledit cadre (1) est constitué d'une matière plastique, en particulier, de polyuréthane, et sa forme s'adapte par adhérence au visage de la personne portant le masque dans la partie devant les yeux, laissant le nez non affecté.

4. Masque à visière selon la revendication 1, dans lequel ladite visière ou ledit verre (2) est un écran curviligne réalisé en une seule pièce à partir d'une matière transparente élastique ou plastique.

5. Masque à visière selon la revendication 1, dans lequel au moins une partie de ladite partie avant du cadre possède une cavité (10) adaptée pour recevoir lesdites parties de type bande ou de type ruban, la partie restante de ladite partie avant ne portant pas de Velcro, de sorte que ladite visière ou ledit verre (2) peut être remplacé facilement.

6. Masque à visière selon la revendication 1, dans lequel ledit bord latéral (5) du cadre possède une structure de type grille (6) avec des ouvertures.

7. Masque à visière selon la revendication 1, dans lequel ladite couche de mousse (3) est constituée d'une matière souple compressible et redilatable, soit en une seule pièce, soit divisée en parties ou zones ayant une épaisseur ou une consistance ou une densité ou des caractéristiques thermiques ou un nombre de couches de composants identiques ou différents.
